# EUROPEAN PATENT APPLICATION

(11) **EP 4 434 436 A1**
(43) Date of publication of application: **25.09.2024**
(21) Application number: 21964734.4
(22) Date of filing: 18.11.2021
(51) Int. Cl.: A61B 1/045

(54) **VIDEO EDITING DEVICE, VIDEO EDITING METHOD, AND RECORDING MEDIUM**

(71) Applicant: NEC Corporation, 108-8001 Tokyo (JP)
(72) Inventor: KAMIJO, Kenichi, Tokyo 108-8001 (JP); OHTSUKA, Shota, Tokyo 108-8001 (JP); KIMURA, Tatsu, Tokyo 108-8001 (JP); MARUGAME, Atsushi, Tokyo 108-8001 (JP); SAIKOU, Masahiro, Tokyo 108-8001 (JP); SHINO, Ryosaku, Tokyo 108-8001 (JP)
(74) Representative: Betten & Resch
(86) International application number: PCT/JP2021/042368
(87) International publication number: WO 2023/089719

(57) **Abstract**

In a video editing device, a video acquisition means acquires an endoscopic video taken by an endoscope. A first timing acquisition means acquires a first timing at which a lesion is detected from the endoscopic video. A second timing acquisition means acquires a second timing at which an examiner instructs photographing based on the endoscopic video. An editing means generates an edited video by extracting, from the endoscopic video, a partial video of a predetermined time period before and after a timing corresponding to at least one of the first timing and the second timing, and output the edited video.

## Description

### TECHNICAL FIELD

The present disclosure relates to editing of a video obtained by an endoscopic examination.

### BACKGROUND ART

The examination image of the endoscope may be reexamined, when the doctor looks back the examination result of the endoscopic examination or when the doctor wants to obtain the opinion of other doctors. However, since the time period of the image is long, the reexamination takes a lot of time. From this viewpoint, Patent Document 1 proposes a method of adding time stamps to the moving image file, during the examination, by the operation of the operator of the endoscope in the endoscopic examination system, thereby to easily retrieve the moving image of the time zone with significant information.

### PRECEDING TECHNICAL REFERENCES

### PATENT DOCUMENT

Patent Document 1: Japanese Patent Application Laid-open under No. JP 2018-166989

### SUMMARY

### PROBLEM TO BE SOLVED

Recently, there have been proposed techniques for detecting lesions using AI (Artificial Intelligence). Therefore, it is desirable to be able to efficiently refer to the examination image including not only the images selected by the operator of the endoscope but also the images of the lesions detected by AI.

It is an object of the present disclosure to create an edited video from an endoscopic examination image, by which necessary parts can be efficiently viewed.

### MEANS FOR SOLVING THE PROBLEM

According to an example aspect of the present disclosure, there is provided a video editing device comprising:
a video acquisition means configured to acquire an endoscopic video taken by an endoscope;
a first timing acquisition means configured to acquire a first timing at which a lesion is detected from the endoscopic video;
a second timing acquisition means configured to acquire a second timing at which an examiner instructs photographing based on the endoscopic video; and
an editing means configured to generate an edited video by extracting, from the endoscopic video, a partial video of a predetermined time period before and after a timing corresponding to at least one of the first timing and the second timing, and output the edited video.

According to another example aspect of the present disclosure, there is provided a video editing method comprising:
acquiring an endoscopic video taken by an endoscope;
acquiring a first timing at which a lesion is detected from the endoscopic video;
acquiring a second timing at which an examiner instructs photographing based on the endoscopic video;
generating an edited video by extracting, from the endoscopic video, a partial video of a predetermined time period before and after a timing corresponding to at least one of the first timing and the second timing; and
outputting the edited video.

According to still another example aspect of the present disclosure, there is provided a recording medium recording a program, the program causing a computer to execute processing of:
acquiring an endoscopic video captured by an endoscope;
acquiring a first timing at which a lesion is detected from the endoscopic video;
acquiring a second timing at which an examiner instructs photographing based on the endoscopic video;
generating an edited video by extracting, from the endoscopic video, a partial video of a predetermined time period before and after a timing corresponding to at least one of the first timing and the second timing; and
outputting the edited video.

### EFFECT

According to the present disclosure, the examination image can be efficiently checked after the endoscopic examination is completed.

### BRIEF DESCRIPTION OF THE DRAWINGS

[FIG. 1] FIG. 1 is a block diagram showing a schematic configuration of an endoscopic examination system.
[FIG. 2] FIG. 2 is a block diagram showing a hardware configuration of an image processing device.
[FIG. 3] FIG. 3 is a block diagram showing a functional configuration of the image processing device.
[FIG. 4] FIG. 4 schematically shows a structure of a large intestine.
[FIG. 5] FIG. 5 is a diagram showing an example of an examination result.
[FIG. 6] FIG. 6 is a diagram showing a method of generating an edited video.
[FIG. 7] FIG. 7 is a display example of reproducing an edited video.
[FIG. 8] FIG. 8 is a flowchart of display processing by the image processing device.
[FIG. 9] FIG. 9 is a display example of the edited video at the start of reproduction.
[FIG. 10] FIG. 10 is a block diagram showing a functional configuration of a video editing device of a second example embodiment.
[FIG. 11] FIG. 11 is a flowchart of processing by the video editing device of the second example embodiment.

### EXAMPLE EMBODIMENTS

Preferred example embodiments of the present disclosure will be described with reference to the accompanying drawings.

### <First example embodiment>

### [System Configuration]

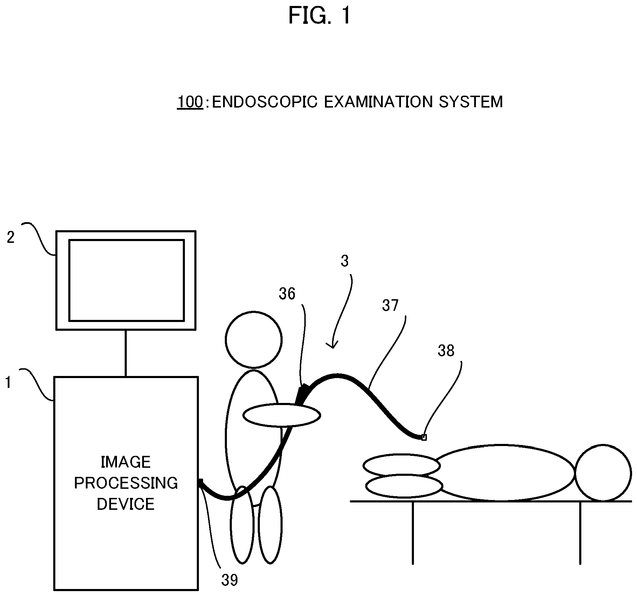

FIG. 1 shows a schematic configuration of an endoscopic examination system 100. When the examination (including the treatment) using the endoscope is performed, the endoscopic examination system 100 acquires the times when the examiner of the endoscopic examination took the still image and the times when the lesions were detected by the image analysis of AI. Then, the endoscopic examination system 100 extracts the partial videos of a predetermined time period before and after the acquired times to generate and display an edited video. This enables the examiner to check the examination video efficiently after the endoscopic examination.

As shown in FIG. 1, the endoscopic examination system 100 mainly includes an image processing device 1, a display device 2, and an endoscope 3 connected to the image processing device 1.

The image processing device 1 acquires a video (i.e., a moving image, hereinafter also referred to as an "endoscopic video Ic") captured by the endoscope 3 during the endoscopic examination from the endoscope 3, and displays display data for the check by the examiner of the endoscopic examination on the display device 2. Specifically, the image processing device 1 acquires a moving image of organs captured by the endoscope 3 as an endoscopic video Ic during the endoscopic examination. In addition, when the examiner finds a lesion during the endoscopic examination, he or she operates the endoscope 3 to input a photographing instruction of the lesion position. Based on the photographing instruction by the examiner, the image processing device 1 generates a lesion image capturing the lesion position. Specifically, the image processing device 1 generates the lesion image which is a still image, from the endoscopic video Ic which is a moving image, on the basis of the photographing instruction of the examiner.

The display device 2 is a display or the like for displaying images on the basis of the display signal supplied from the image processing device 1.

The endoscope 3 mainly includes an operation unit 36 used by the examiner to input instructions such as air supply, water supply, angle adjustment, and the photographing instruction, a shaft 37 having flexibility and inserted into an organ of a subject to be examined, a tip portion 38 with a built-in image-taking unit such as an ultra-compact imaging element, and a connection unit 39 for connection with the image processing device 1.

While the following explanation is mainly given on the processing of endoscopic examination for a large intestine, the subjects of examination may be gastrointestinal (digestive organs) such as the stomach, esophagus, small intestine, and duodenum, as well as the large intestine.

In addition, the part to be detected in the endoscopic examination is not limited to the lesion part, but may be any part (also referred to as a "region of interest") in which the examiner needs attention. The region of interest may be, for example, a lesion part, a part where inflammation has occurred, a part where a surgical scar or other cut has occurred, a part where a fold or protrusion has occurred, or a part where the tip portion 38 of the endoscope 3 is likely to contact (easily stuck) the intraluminal wall surface.

### [Hardware configuration]

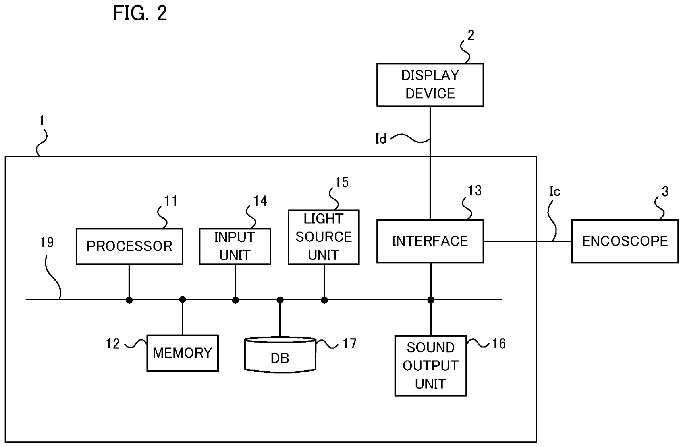

FIG. 2 shows a hardware configuration of the image processing device 1. The image processing device 1 mainly includes a processor 11, a memory 12, an interface 13, an input unit 14, a light source unit 15, a sound output unit 16, and a data base (hereinafter referred to as "DB") 17. These elements are connected with each other via a data bus 19.

The processor 11 executes a predetermined processing by executing a program stored in the memory 12. The processor 11 is a processor such as a CPU (Central Processing Unit), a GPU (Graphics Processing Unit), and a TPU (Tensor Processing Unit). The processor 11 may be configured by multiple processors. The processor 11 is an example of a computer.

The memory 12 is configured by various volatile memories used as a working memory and non-volatile memories for storing information needed for the processing of the image processing device 1, such as a RAM (Random Access Memory) and a ROM (Read Only Memory). Incidentally, the memory 12 may include an external storage device such as a hard disk connected to or incorporated in the image processing device 1, and may include a storage medium such as a removable flash memory or a disk medium. The memory 12 stores a program for the image processing device 1 to execute processing in the present example embodiment.

Also, the memory 12 temporarily stores a series of endoscopic videos Ic taken by the endoscope 3 during the endoscopic examination, based on the control of the processor 11. Further, the memory 12 temporarily stores the lesion images photographed in response to the photographing instructions by the examiner during the endoscopic examination. These images are stored in the memory 12 in association with, for example, subject identification information (e.g., the patient ID) and time stamp information, etc.

The interface 13 performs an interface operation between the image processing device 1 and the external devices. For example, the interface 13 supplies the display data Id generated by the processor 11 to the display device 2. Also, the interface 13 supplies the illumination light generated by the light source unit 15 to the endoscope 3. Further, the interface 13 supplies an electrical signal indicating the endoscopic video Ic supplied from the endoscope 3 to the processor 11. The interface 13 may be a communication interface such as a network adapter for wired or wireless communication with an external device, or may be a hardware interface compliant with a USB (Universal Serial Bus), SATA (Serial Advanced Technology Attachment), etc.

The input unit 14 generates an input signal based on the operation of the examiner. The input unit 14 is, for example, a button, a touch panel, a remote controller, a voice input device, or the like. The light source unit 15 generates the light to be delivered to the tip portion 38 of the endoscope 3. The light source unit 15 may also incorporate a pump or the like for delivering water or air to be supplied to the endoscope 3. The sound output unit 16 outputs the sound based on the control of the processor 11.

The DB 17 stores the endoscopic videos acquired by the past endoscopic examinations of the subject, and the lesion information. The lesion information includes lesion images and associated information. The DB 17 may include an external storage device, such as a hard disk connected to or incorporated in the image processing device 1, and may include a storage medium, such as a removable flash memory. Instead of providing the DB 17 in the endoscopic examination system 100, the DB 17 may be provided in an external server or the like to acquire associated information from the server through communication.

### [Functional configuration]

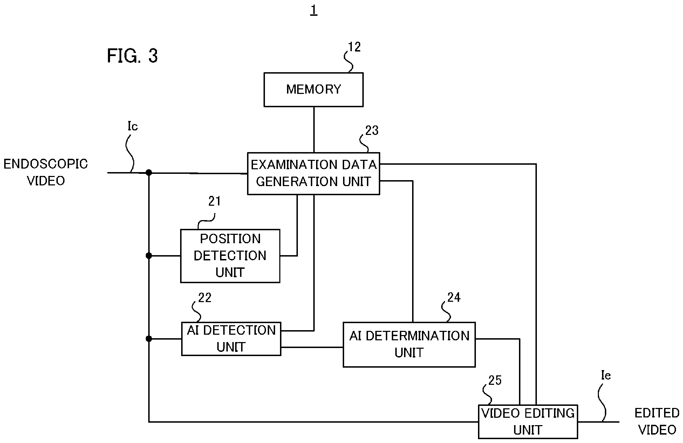

FIG. 3 is a block diagram showing a functional configuration of the image processing device 1. The image processing device 1 functionally includes a position detection unit 21, an AI detection unit 22, an examination data generation unit 23, an AI determination unit 24, and a video editing unit 25.

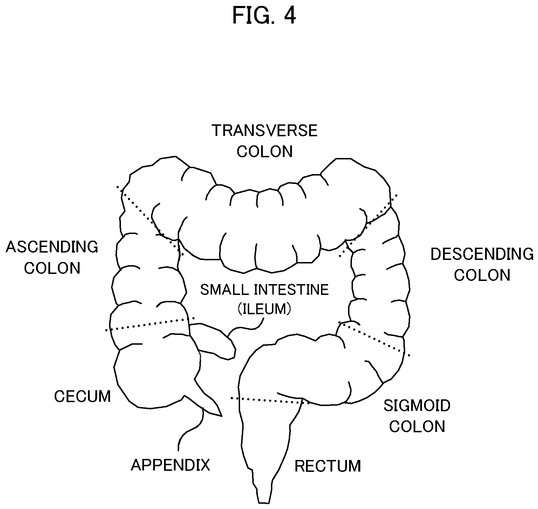

The image processing device 1 receives the endoscopic video Ic from the endoscope 3. The endoscopic video Ic is inputted into the position detection unit 21, the AI detection unit 22, the examination data generation unit 23, and the video editing unit 25. The position detection unit 21 detects the position of the endoscope 3, i.e., the imaging position of the endoscopic video, based on the endoscopic video Ic. Specifically, the position detection unit 21 detects the imaging position by image analysis of the inputted endoscopic video Ic. Here, the imaging position may be three-dimensional coordinates in the organ of the examination target, but may be at least an information indicating one of a plurality of regions in the organ of the examination target. For example, as shown in FIG. 4, the large intestine includes multiple regions (sites) such as the cecum, the ascending colon, the transverse colon, the descending colon, the sigmoid colon, and the rectum. Therefore, when the examination target is the large intestine, the position detection unit 21 may detect whether at least the imaging position belongs to any of the above-described regions.

Specifically, the position detection unit 21 can estimate which region of the large intestine the imaging position at that time belongs to, based on the pattern of the mucous membrane, the presence or absence of the folds, the shape of the folds in the endoscopic video, the number of the folds passed by the movement of the endoscope 3, and the like. The position detection unit 21 may estimate the imaging position by estimating the movement speed of the endoscope 3 based on the endoscopic video and calculating the movement distance in the large intestine based on the movement speed and the time. In addition to the image analysis of the endoscopic video, the position detection unit 21 may detect the imaging position using the insertion length of the endoscope 3 inserted into the organ. In the present example embodiment, the detection method of the imaging position in the organ of the examination target is not limited to a specific method. The position detection unit 21 outputs the detected imaging position to the examination data generation unit 23.

The AI detection unit 22 performs image analysis based on the endoscopic video Ic to determine whether or not lesions exist. The AI detection unit 22 detects lesion-like parts included in the endoscopic video using an image recognition model prepared in advance. When detecting the lesion-like part, the AI detection unit 22 generates a lesion image which is a still image. Further, the AI detection unit 22 acquires the time information at the time when it detects the lesion-like part. The AI detection unit 22 generates AI detection data including the lesion images and the time information, and outputs the AI detection data to the examination data generation unit 23 and the AI determination unit 24. On the other hand, when the AI determination unit does not detect any lesion-like part, it outputs the determination result indicating that there is no lesion.

Further, to the image processing device 1, patient information of the patient who is a subject is inputted through the input unit 14. The patient information is information that uniquely identifies a patient, and may be a personal identification number such as My Number in addition to the patient name and/or the ID uniquely assigned to each patient. The patient information is inputted to the examination data generation unit 23.

The examination data generation unit 23 generates a still image based on the photographing instruction of the examiner during the endoscopic examination. Further, the examination data generation unit 23 acquires the time information at the time when the examiner made the photographing instruction. The examination data generating unit 23 generates examination data including the endoscopic video Ic, the photographing position detected by the position detection unit 21, the patient information, the AI detection data generated by the AI detection unit 22, the still image taken based on the examiner's photographing instruction, and the time information at the time when the examiner made the photographing instruction, and stores the examination data in the memory 12. Further, the examination data generation unit 23 outputs the generated examination data to the AI determination unit 24 and the video editing unit 25.

The AI determination unit 24 acquires the still image taken on the basis of the photographing instruction of the examiner and the still image of the lesion-like part detected by the AI from the AI detection data generated by the AI detection unit 22 and the examination data generated by the examination data generation unit 23. Then, the AI determination unit 24 performs image analysis on the acquired still images, and performs qualitative determination to determine whether the lesion-like part is neoplastic or non-neoplastic. The AI determination unit 24 outputs the result of the qualitative determination to the video editing unit 25. Incidentally, the still images taken based on the photographing instruction of the examiner may include the still images in which the lesion-like part is not captured, such as the still images of the residue, in addition to the still images in which the lesion-like part is captured. When the AI determination unit 24 does not detect the lesion-like part in the still image captured based on the photographing instruction of the examiner, it does not perform the qualitative determination and outputs the determination result indicating that there is no lesion.

The video editing unit 25 generates the video data Ie using the endoscopic video Ic, the examination data inputted from the examination data generation unit 23, and the result of the quality determination inputted from the AI determination unit 24, and outputs the generated video data Ie to the display device 2.

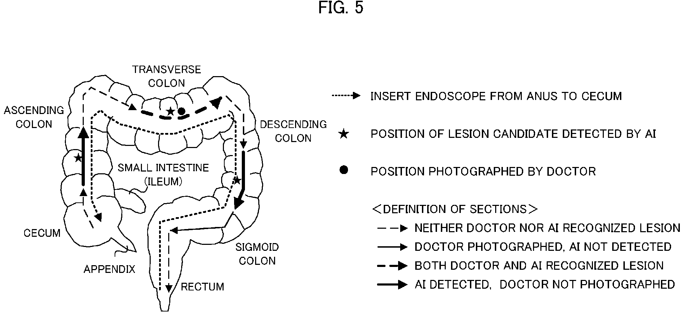

FIG. 5 shows an example of the result of the endoscopic examination of a patient. FIG. 5 shows a model image of the large intestine for convenience. The model image is an image of a schematic diagram of the large intestine, on which the examination result is superimposed. Here, as the examination result, the result of inserting the endoscope from the anus to the cecum, the positions of the lesion candidate parts detected by the AI, the position photographed by the doctor, and the type of the sections are displayed. Specifically, the result of inserting the endoscope from the anus to the cecum is indicated by the dotted line. Also, the star marks indicate the positions where the AI has detected the lesion candidate part, and the black circle indicates the position photographed by the doctor. Then, the endoscopic video is classified into a plurality of sections, and the plurality of sections are distinguished and displayed by the thickness of the line and the type of the line. Here, the sections where neither the doctor nor the AI recognized the lesion (the cecum and the rectum in this example) are indicated by the thin broken line. The section where the doctor photographed but the AI did not detect the lesion (the sigmoid colon in this case) is indicated by the thin solid line. The section where both the doctor and the AI recognized the lesion (the transverse colon in this case) is shown with thick dashed line. The sections where the AI detected the lesion but the doctor did not photograph (the ascending colon and the descending colon in this case) are indicated by the thick solid lines.

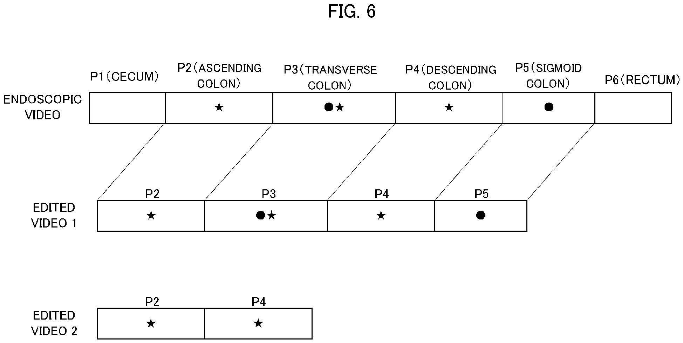

FIG. 6 shows a method of editing video by the video editing unit 25. The video editing unit 25 generates an edited video based on the endoscopic video Ic and the examination data generated by the examination data generation unit 23. In one example, the video editing unit 25 extracts the partial videos of a predetermined time period before and after the time when the doctor instructed the photographing or the AI detected the lesion-like part from the endoscopic video Ic, and generates the edited video 1. Here, the video editing unit 25 extracts the sections (P2 to P5 in this example) including the position where the doctor instructed the photographing (circle) or the position where the AI detected the lesion-like part (star mark) from the sections included in the captured video (P1 to P6), and generates the edited video 1 by concatenating those sections in a time series.

The section from which the partial video is extracted is not limited to the above example. In another example, the video editing unit 25 may extract the sections including the position where the AI detected the lesion candidate part but the doctor did not instruct the photographing (P2, P4 in this example), and generate the edited video 2. In yet another example, the video editing unit 25 may generate the edited video based on the qualitative determination by the AI determination unit 24. For example, the video editing unit 25 may extract only the sections including the position where the AI determination unit 24 performed the qualitative determination to generate the edited video. Alternatively, the video editing unit 25 may extract only the sections including the lesion determined to be neoplastic by the the qualitative determination by the AI determination unit 24 and generate the edited video. In this way, the examiner can efficiently review the examination result by viewing the edited video after the endoscopic examination.

### [Display example]

Next, a display example by the display device 2 will be described.

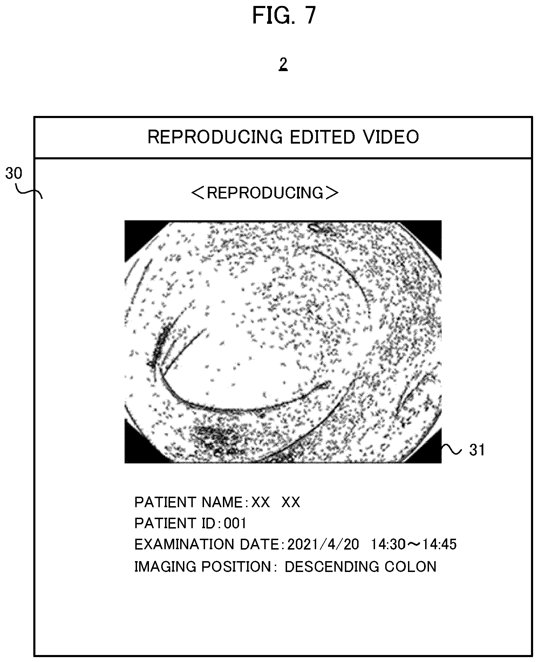

FIG. 7 shows a display example of the edited video. FIG. 7 is a display example of the display device 2 when the edited video generated by the video editing unit 25 is reproduced as described above. In the display example of FIG. 7, the video 31 being reproduced is displayed in the display area 30, in addition to the information such as the patient name, the patient ID, the examination date, and the current imaging position. For example, when the edited video 1 shown in FIG. 6 is being reproduced, the videos of the sections P2 to P5 are reproduced in this order as the video 31. Further, when the section being reproduced as the video 31 changes, the imaging position changes. Thus, the examiner can efficiently check significant positions in the endoscopic video by watching the edited video.

### [Display processing]

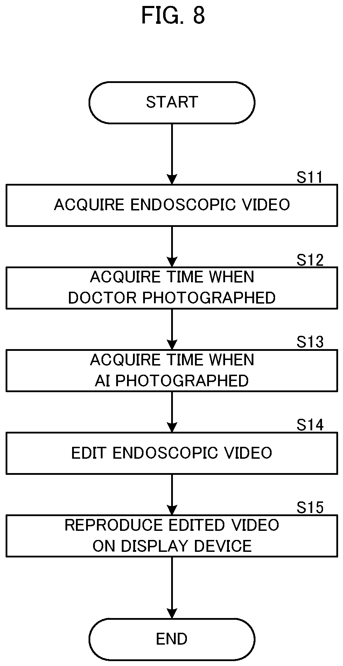

Next, display processing for performing the above-mentioned display will be described. FIG. 8 is a flowchart of display processing by the image processing device 1. This processing is realized by the processor 11 shown in FIG. 2, which executes a pre-prepared program and operates as each element shown in FIG. 3.

First, the examination data generation unit 23, the AI detection unit 22, and the video editing unit 25 acquire the endoscopic video Ic (step S11). Next, the examination data generation unit 23 acquires the time when the doctor instructs the photographing (step S12). The AI detection unit 22 acquires the time of detecting the lesion-like part (step S13). Next, the video editing unit 25 extracts the partial videos of a predetermined time period before and after the time when the examiner instructed the photographing and the time when the AI detection unit 22 detected the lesion-like part from the endoscopic video Ic. Then, the video editing unit 25 generates the edited video by editing the endoscopic video Ic (step S14) and outputs it to the display device 2. The displaying device 2 reproduces the received edited video (step S15). Thus, the edited video is reproduced as shown in FIG. 7.

### [Modifications]

### (Modification 1)

In the above-described example embodiment, as illustrated in FIG. 6, the video editing unit 25 cuts the video of the section where neither the doctor nor the AI recognizes the lesion to create the edited video. Instead, the video editing unit 25 may edit the video such that the section where neither the doctor nor the AI recognizes the lesion is not cut, but is reproduced at a high speed. In this case, when the edited video is reproduced, the section where the doctor or the AI recognized the lesion is reproduced at the normal speed, and the section where neither the doctor nor the AI recognized the lesion is reproduced at the higher speed than the normal speed. Therefore, the examiner or the like can take a quick look of the section where neither the doctor nor the AI recognizes the lesion at high speed and carefully observe the section where the doctor or the AI recognized the lesion at the normal speed.

### (Modification 2)

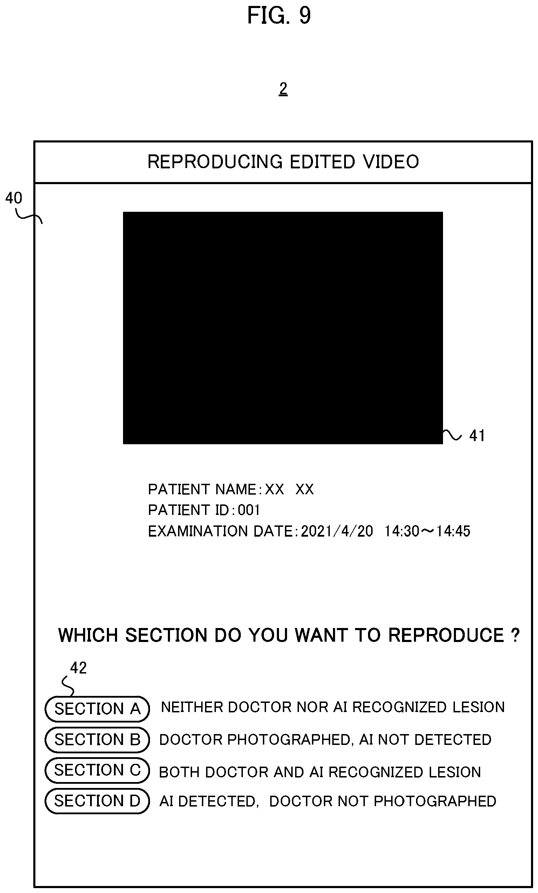

In the above-described example embodiment, the video of a predetermined condition, such as the timing when the examiner instructed photographing or the timing when the AI detected the lesion-like part, is displayed as the edited video. However, the application of the present disclosure is not limited thereto. For example, the user may select the sections to be reproduced as shown in FIG. 9.

FIG. 9 shows a display example at the start of reproducing the edited video. Specifically, in FIG. 9, the image display area 41 and the section buttons 42 are displayed in the display area 40, in addition to the basic information such as the patient name, the patient ID and the examination date. The endoscope video is divided into the section where neither the doctor nor the AI recognized the lesion (section A), the section where the doctor photographed but the AI did not detect the lesion (section B), the section where the doctor and the AI recognized the lesion (section C), and the section where the AI detected the lesion but the doctor did not photograph (section D). When the user presses the section button 42 of the section that he or she wants to reproduce from among these sections, the video of the selected section is displayed on the video display area 41. Thus, the user can easily display a digest video of the sections that he or she wants to check.

### (Modification 3)

While the above example embodiment uses an endoscope of the type in which a shaft is inserted to directly observe the gastrointestinal tract, a capsule endoscope may be used instead. When the capsule endoscope is used to detect the lesion, the doctor can efficiently check the video by extracting the partial video of a predetermined time period before and after the time point of detecting the lesion.

### <Second example embodiment>

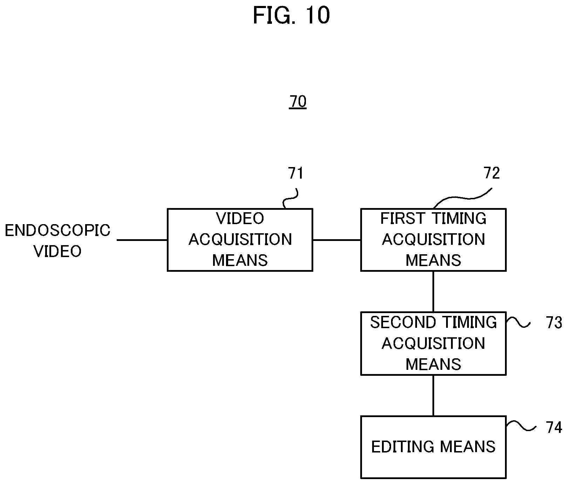

FIG. 10 is a block diagram showing a functional configuration of a video editing device according to a second example embodiment. The video editing device 70 includes a video acquisition means 71, a first timing acquisition means 72, a second timing acquisition means 73, and an editing means 74.

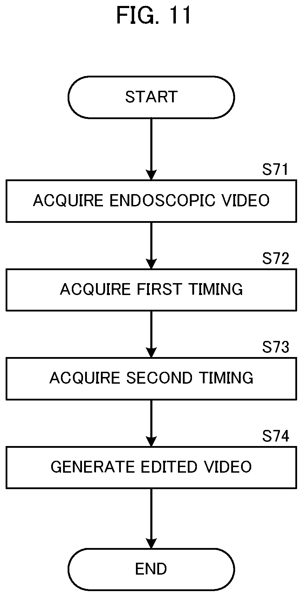

FIG. 11 is a flowchart illustrating processing performed by the video editing device according to the second example embodiment. The video acquisition means 71 acquires an endoscopic video taken by an endoscope (step S71). The first timing acquisition means 72 acquires a first timing at which a lesion is detected from the endoscopic video (step S72). The second timing acquisition means 73 acquires a second timing at which an examiner instructs photographing based on the endoscopic video (step S73). The editing means 74 generates an edited video by extracting, from the endoscopic video, a partial video of a predetermined time period before and after a timing corresponding to at least one of the first timing and the second timing, and output the edited video (step S74).

According to the video editing device 70 of the second example embodiment, it is possible to generate an edited video efficiently showing a required portion from the examination video of the endoscopic examination.

A part or all of the above example embodiments may also be described as in the following supplementary notes, but are not limited to:

### (Supplementary note 1)

A video editing device comprising:
a video acquisition means configured to acquire an endoscopic video taken by an endoscope;
a first timing acquisition means configured to acquire a first timing at which a lesion is detected from the endoscopic video;
a second timing acquisition means configured to acquire a second timing at which an examiner instructs photographing based on the endoscopic video; and
an editing means configured to generate an edited video by extracting, from the endoscopic video, a partial video of a predetermined time period before and after a timing corresponding to at least one of the first timing and the second timing, and output the edited video.

### (Supplementary note 2)

The video editing device according to claim 1, wherein the editing means generates the edited video by extracting the partial video for the timing corresponding to the first timing and not corresponding to the second timing.

### (Supplementary note 3)

The video editing device according to claim 1, further comprising a determination means configured to perform qualitative determination for the partial video of the first timing and the second timing,
wherein the editing means generates the edited video by extracting the partial video on which the qualitative determination is performed.

### (Supplementary note 4)

The video editing device according to claim 1, further comprising a display means configured to divide the endoscopic video into plural types of sections based on whether or not the endoscopic video corresponds to the first timing and the second timing, and display the plural of types of sections as options,
wherein the editing means generates the edited video by extracting the partial video belonging to the section of the type selected by a user from among the plural types of sections.

### (Supplementary note 5)

The video editing device according to any one of claims 1 to 4, wherein the editing means generates the edited video by concatenating the partial videos in a time series.

### (Supplementary note 6)

The video editing device according to any one of claims 1 to 4, wherein the editing means generates the edited video in which the partial video is reproduced at a normal speed and a part of the endoscopic video other than the partial video is reproduced at a higher speed than the normal speed.

### (Supplementary note 7)

A video editing method comprising:
acquiring an endoscopic video taken by an endoscope;
acquiring a first timing at which a lesion is detected from the endoscopic video;
acquiring a second timing at which an examiner instructs photographing based on the endoscopic video;
generating an edited video by extracting, from the endoscopic video, a partial video of a predetermined time period before and after a timing corresponding to at least one of the first timing and the second timing; and
outputting the edited video.

### (Supplementary note 8)

A recording medium recording a program, the program causing a computer to execute processing of:
acquiring an endoscopic video captured by an endoscope;
acquiring a first timing at which a lesion is detected from the endoscopic video;
acquiring a second timing at which an examiner instructs photographing based on the endoscopic video;
generating an edited video by extracting, from the endoscopic video, a partial video of a predetermined time period before and after a timing corresponding to at least one of the first timing and the second timing; and
outputting the edited video.

While the present disclosure has been described with reference to the example embodiments and examples, the present disclosure is not limited to the above example embodiments and examples. Various changes which can be understood by those skilled in the art within the scope of the present disclosure can be made in the configuration and details of the present disclosure.

### DESCRIPTION OF SYMBOLS

1 Image processing device
2 Display device
3 Endoscope
11 Processor
12 Memory
17 Database (DB)
21 Position detection unit
22 AI detection unit
23 Examination data generation unit
24 AI determination unit
25 Video editing unit
100 Endoscopic examination system

## Claims

1. A video editing device comprising:
a video acquisition means configured to acquire an endoscopic video taken by an endoscope;
a first timing acquisition means configured to acquire a first timing at which a lesion is detected from the endoscopic video;
a second timing acquisition means configured to acquire a second timing at which an examiner instructs photographing based on the endoscopic video; and
an editing means configured to generate an edited video by extracting, from the endoscopic video, a partial video of a predetermined time period before and after a timing corresponding to at least one of the first timing and the second timing, and output the edited video.

2. The video editing device according to claim 1, wherein the editing means generates the edited video by extracting the partial video for the timing corresponding to the first timing and not corresponding to the second timing.

3. The video editing device according to claim 1, further comprising a determination means configured to perform qualitative determination for the partial video of the first timing and the second timing,
wherein the editing means generates the edited video by extracting the partial video on which the qualitative determination is performed.

4. The video editing device according to claim 1, further comprising a display means configured to divide the endoscopic video into plural types of sections based on whether or not the endoscopic video corresponds to the first timing and the second timing, and display the plural of types of sections as options,
wherein the editing means generates the edited video by extracting the partial video belonging to the section of the type selected by a user from among the plural types of sections.

5. The video editing device according to any one of claims 1 to 4, wherein the editing means generates the edited video by concatenating the partial videos in a time series.

6. The video editing device according to any one of claims 1 to 4, wherein the editing means generates the edited video in which the partial video is reproduced at a normal speed and a part of the endoscopic video other than the partial video is reproduced at a higher speed than the normal speed.

7. A video editing method comprising:
acquiring an endoscopic video taken by an endoscope;
acquiring a first timing at which a lesion is detected from the endoscopic video;
acquiring a second timing at which an examiner instructs photographing based on the endoscopic video;
generating an edited video by extracting, from the endoscopic video, a partial video of a predetermined time period before and after a timing corresponding to at least one of the first timing and the second timing; and
outputting the edited video.

8. A recording medium recording a program, the program causing a computer to execute processing of:
acquiring an endoscopic video captured by an endoscope;
acquiring a first timing at which a lesion is detected from the endoscopic video;
acquiring a second timing at which an examiner instructs photographing based on the endoscopic video;
generating an edited video by extracting, from the endoscopic video, a partial video of a predetermined time period before and after a timing corresponding to at least one of the first timing and the second timing; and
outputting the edited video.
